# FASCICULE DE BREVET EUROPEEN

(11) **EP 1 933 888 B1**
(45) Date de publication et mention de la délivrance du brevet: **04.03.2015**
(21) Numéro de dépôt: 05824592.9
(22) Date de dépôt: 05.12.2005
(51) Int. Cl.: A61L 2/24, A61L 2/26, A61L 2/10, G06F 19/00

(54) **SYSTEME DE DESINFECTION D'INSTRUMENTS MÉDICAUX**
DESINFEKTIONSSYSTEM FÜR MEDIZINISCHE INSTRUMENTE
MEDICAL INSTRUMENT DISINFECTING SYSTEM

(30) Priorité: 15.09.2005 FR 0509452
(43) Date de publication de la demande: 25.06.2008
(73) Titulaire: Germitec, 92110 Clichy (FR)
(72) Inventeur: DESHAYS, Clément, F-07120 Ruoms (FR)
(74) Mandataire: Jacobson, Claude
(86) Numéro de dépôt international: PCT/FR2005/003032
(87) Numéro de publication internationale: WO 2007/031613

(56) Documents cités:
- EP-A- 0 471 530
- EP-A- 0 630 820
- EP-A- 1 402 904
- US-A- 5 310 524
- US-A1- 2001 024 623
- US-A1- 2003 039 579
- US-A1- 2003 187 586
- US-B1- 6 485 979

## Description

La présente invention concerne un système de désinfection d'instruments médicaux.

On connaît déjà dans l'état de la technique, des systèmes de désinfection de ce type qui comportent par exemple une enceinte de désinfection adaptée pour mettre en oeuvre un cycle de désinfection des instruments.

Ces enceintes peuvent par exemple être des enceintes associées à des moyens d'émission d'un rayonnement UV du type UVC par exemple ou à des moyens de désinfection chimiques, etc...

A titre d'exemple, on pourra se reporter au document EP-A-0839537 qui décrit un dispositif de support d'instruments dans une enceinte notamment de décontamination et une enceinte correspondante.

En fait ce document se rapporte à une enceinte de décontamination pour instruments médicaux délimitée par un fond, au moins une paroi latérale et un couvercle supérieur, chaque instrument comportant une partie active et une partie de raccordement sous forme d'un câble.

Cette enceinte comporte également une potence s'étendant à l'intérieur et en partie supérieure de l'enceinte, parallèlement au fond et en surplomb du fond, cette potence comportant une pluralité d'organes de suspension, chacun d'eux étant destiné à coopérer avec une partie du câble voisine de la partie active de l'instrument.

Cette enceinte est également associée à des moyens par exemple à tubes d'émission dans celle-ci d'un rayonnement UV de type C par exemple pour assurer la désinfection des instruments.

Bien entendu, d'autres moyens de désinfection peuvent être envisagés.

Cependant, tous les systèmes de l'état de la technique posent un certain nombre de problèmes notamment au niveau de la traçabilité de la désinfection des instruments.

Par exemple, le document US 6 485 979 décrit un système de suivi d'instruments médicaux dans lequel l'état de désinfection d'un instrument est vérifié en utilisant une étiquette à usage unique, qui change d'état au cours de la désinfection. Un autre système de suivi d'instruments médicaux est connu par le document EP-A-0630820.

Le but de l'invention est donc de résoudre ces problèmes.

A cet effet, l'invention a pour objet un système de désinfection d'instruments médicaux tel que défini dans la revendication 3.

Selon des modes particuliers de réalisation, le système selon l'invention comporte une ou plusieurs des caractéristiques définies dans les revendications dépendantes 4 à 9

L'invention a également pour objet un procédé de désinfection tel que défini dans la revendication dépendante 1.

Selon un mode particulier de réalisation, le procédé selon l'invention comporte la caractéristique définie dans la revendication dépendante 2.

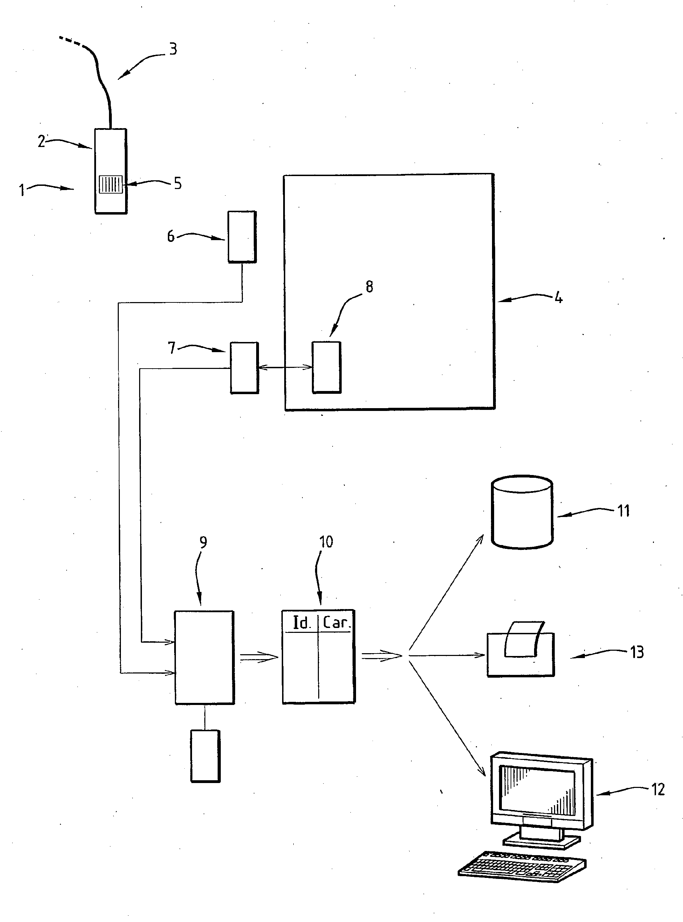

L'invention sera mieux comprise à l'aide de la description qui va suivre, donnée uniquement à titre d'exemple et faite en se référant au dessin annexé qui représente un schéma synoptique illustrant la structure et le fonctionnement d'un système de désinfection selon l'invention.

On a en effet illustré sur cette figure, un système de désinfection d'instruments médicaux.

Un tel instrument est par exemple désigné par la référence générale 1 sur cette figure et se présente par exemple sous la forme d'une sonde de type sonde échographique ou autre.

Cette sonde comporte alors une partie active désignée par la référence générale 2 et un câble de raccordement désigné par la référence générale 3.

Cette sonde est adaptée pour être mise en place et retirée d'une enceinte de désinfection désignée par la référence générale 4 sur la figure. Cette enceinte de désinfection étant adaptée pour mettre en oeuvre un cycle de désinfection des instruments.

Comme cela a été mentionné précédemment, différents types d'enceintes et différents types de cycles de désinfection peuvent être envisagés que ce soit par exemple par rayonnement, chimique ou autre.

Dans le système selon l'invention, chaque instrument porte des informations d'identification de celui-ci.

A titre d'exemple, ces informations d'identification peuvent être constituées par un code à barre désigné par la référence générale 5 sur la figure, ce code à barre étant par exemple porté par la partie active ou encore par le câble de raccordement de l'instrument.

Bien entendu, d'autres modes de réalisation peuvent être envisagés.

L'enceinte est alors associée à des moyens d'acquisition de ces informations d'identification de chaque instrument.

Ces moyens d'acquisition de ces informations d'identification sont désignés par la référence générale 6 sur cette figure et comprennent par exemple tout capteur approprié tel que par exemple un lecteur de code à barre ou autre.

Ce capteur est alors adapté pour acquérir les informations d'identification du ou de chaque instrument lors de sa mise en place et de son retrait de l'enceinte au début et à la fin d'un cycle de désinfection.

A titre d'exemple, ces moyens d'acquisition peuvent se présenter sous la forme d'un capteur extérieur à l'enceinte se présentant par exemple sous la forme d'un capteur de type « douchette » ou encore sous la forme d'un capteur directement intégré dans l'enceinte de désinfection par exemple sur la potence de l'enceinte décrite dans le document EP-A-0839537 mentionné précédemment.

De plus, l'enceinte est associée à des moyens d'acquisition d'informations de caractérisation du cycle de décontamination, c'est-à-dire plus particulièrement des conditions de son déroulement.

Ces moyens sont désignés par la référence générale 7 sur cette figure et peuvent comporter différents types de moyens d'acquisition d'informations adaptés pour acquérir des informations choisies dans un groupe d'informations comprenant par exemple des information d'identification de l'enceinte, chaque enceinte étant alors affecté d'un numéro d'identification spécifique stocké dans celle-ci, des information d'horodatage du cycle permettant par exemple d'acquérir la date du cycle, le numéro journalier du cycle, l'heure de début et l'heure de fin du cycle, à partir d'un circuit formant horloge, etc.

Ces informations de caractérisation comportent également des informations relatives à la dose d'UV émise lors d'un cycle l'enceinte de désinfection étant munie de moyens de génération de rayonnement UV de désinfection.

Ces informations sont alors déterminées à partir d'un capteur de tout type approprié déjà connu dans l'état de la technique et désigné par exemple par la référence générale 8 sur cette figure.

Ce capteur peut alors être par exemple implanté sous la potence de l'enceinte décrite dans le document EP cité précédemment.

Ces différentes informations, c'est-à-dire les informations d'identification du ou de chaque instrument et les informations de caractérisation du cycle de désinfection, sont alors transmises à une unité de traitement d'informations désignée par la référence générale 9 sur cette figure et constituée par tout calculateur approprié, par exemple intégré dans les moyens de pilotage du fonctionnement de l'enceinte, pour mettre en oeuvre une fonction d'association de ces informations afin d'engendrer des informations de traçabilité de la désinfection.

En effet, cette unité de traitement d'information 9 est adaptée pour associer les informations d'identification du ou de chaque instrument présent dans l'enceinte lors d'un cycle de désinfection avec les informations de caractérisation du déroulement de ce cycle, afin de délivrer des informations du déroulement de ce cycle, afin de délivrer des informations permettant d'assurer la traçabilité de la désinfection du ou de chaque instrument.

Ces informations de traçabilité sont désignées par la référence générale 10 sur la figure et permettent donc de mettre en relation chaque instrument avec les conditions dans lesquelles s'est déroulé le cycle de désinfection correspondant.

Il est à noter que ces informations de traçabilité ne peuvent être émises que si un instrument a bien été identifié lors de son introduction dans l'enceinte avant le début du cycle et lors de son retrait de cette enceinte après la fin de ce cycle.

L'opérateur doit donc alors obligatoirement identifier l'instrument lors de sa mise en place et de son retrait de l'enceinte. Dans le cas contraire, l'unité de traitement d'informations n'engendre pas les informations de traçabilité.

Ces informations de traçabilité sont alors disponibles pour assurer la traçabilité de l'opération de désinfection en vue par exemple d'un stockage de ces informations dans des moyens de stockage d'informations comme cela est illustré en 11 sur cette figure, d'une visualisation de celles-ci par exemple sur un afficheur quelconque désigné par la référence générale 12, ou encore d'une impression de celles-ci par exemple à l'aide de moyens d'impression quelconques telle qu'une imprimante désignée par la référence générale 13.

On notera par exemple qu'une telle imprimante peut être adaptée pour imprimer les informations de traçabilité sur un autocollant pouvant être associé par exemple à un dossier de patient ayant été en contact avec l'instrument désinfecté, un registre de traçabilité, etc...

A titre d'exemple, les informations de caractérisation portées par cet autocollant comportent alors une information de dose d'UV reçue par l'instrument lors de son passage dans l'enceinte de désinfection, cette dose étant par exemple déterminée à partir de la puissance ou éclairement UV émis lors du cycle multiplié par la durée de ce cycle.

On sait en effet que ce paramètre peut être déterminant pour obtenir tel ou tel niveau de désinfection des instruments.

On conçoit alors qu'un tel système permet d'assurer une traçabilité optimale de la désinfection d'instruments de ce type dans la mesure où les informations de traçabilité permettent de garantir le passage de l'instrument dans l'enceinte et de vérifier les informations de caractérisation du cycle de désinfection subi par l'instrument, c'est-à-dire notamment le moment où cette désinfection a eu lieu, l'enceinte dans laquelle s'est déroulé le cycle de désinfection et la dose notamment d'UV reçue par l'instrument.

Bien entendu d'autres modes de réalisation peuvent encore être envisagés.

## Revendications

1. Procédé de désinfection d'instruments médicaux (1) dans une enceinte (4) de désinfection comportant des moyens de génération d'un rayonnement UV de désinfection des instruments et un capteur correspondant (8) implanté, adaptée pour mettre en oeuvre un cycle de désinfection des instruments, **caractérisé en ce que** chaque instrument (1) comporte des informations d'identification (5) et **en ce que** le procédé comporte les étapes suivantes :
- acquérir des informations d'identification du ou de chaque instrument (1) lors de sa mise en place et de son retrait de l'enceinte (4) au début et à la fin d'un cycle de désinfection ;
- acquérir des informations de caractérisation du cycle de désinfection, les informations de caractérisation du cycle de désinfection comprenant des informations de dose d'UV émise lors du cycle; et
- associer des informations d'identification du ou de chaque instrument et des informations de caractérisation du cycle de désinfection pour engendrer des informations de traçabilité de la désinfection du ou de chaque instrument, lesdites informations de traçabilité n'étant émises que si l'instrument correspondant (1) a bien été identifié lors de sa mise en place et de son retrait de l'enceinte (4) avant et après le cycle de désinfection, respectivement.

2. Procédé de désinfection selon la revendication 1, **caractérisé en ce que** les informations d'identification sont acquises par un capteur directement intégré dans l'enceinte de désinfection.

3. Système de désinfection d'instruments médicaux (1) du type comportant une enceinte (4) de désinfection adaptée pour mettre en oeuvre un cycle de désinfection des instruments selon un procédé selon la revendication 1 ou 2, comportant des moyens de génération d'un rayonnement UV de désinfection des instruments, **caractérisé en ce que** chaque instrument (1) comporte des informations d'identification (5) et **en ce que** l'enceinte (4) est associée à des moyens (6) d'acquisition des informations d'identification du ou de chaque instrument (1) lors de sa mise en place et de son retrait de l'enceinte (4) au début et à la fin d'un cycle de désinfection, à des moyens (7, 8) d'acquisition d'informations de caractérisation du cycle de désinfection comprenant des informations de dose d'UV émise lors du cycle, issues d'un capteur correspondant (8) implanté dans l'enceinte, et à des moyens (9, 10, 11, 12, 13) d'association des informations d'identification du ou de chaque instrument et des informations de caractérisation du cycle de désinfection pour engendrer des informations de traçabilité de la désinfection du ou de chaque instrument et **en ce que** les moyens (9) d'association des informations sont adaptés pour n'émettre les informations de traçabilité que si l'instrument correspondant (1) a bien été identifié lors de sa mise en place et de son retrait de l'enceinte (4) avant et après le cycle de désinfection, respectivement.

4. Système de désinfection selon la revendication 2, **caractérisé en ce que** les informations d'identification (5) de chaque instrument se présentent sous la forme d'un code à barre et **en ce que** les moyens d'acquisition correspondants (6) de l'enceinte (4) comprennent un lecteur de code.

5. Système de désinfection selon la revendication 4, **caractérisé en ce que** l'enceinte (4) comporte une potence de suspension des instruments et **en ce que** le lecteur (6) est fixé sur cette potence.

6. Système de désinfection selon l'une quelconque des revendications 3 à 5, **caractérisé en ce que** les moyens (7, 8) d'acquisition d'informations de caractérisation du cycle de désinfection comprennent des moyens d'acquisition d'informations choisies dans le groupe d'informations comprenant des informations d'identification de l'enceinte (4) et des informations d'horodatage du cycle de désinfection.

7. Système de désinfection selon la revendication 5, **caractérisé en ce que** en ce que le capteur d'UV (8) est implanté sous la potence de l'enceinte.

8. Système de désinfection selon l'une quelconque des revendications 3 à 7, **caractérisé en ce que** les moyens (9) d'association des informations sont associés à des moyens de visualisation (12) de celles-ci, de stockage (11) de celles-ci et / ou d'impression (13) de celles-ci.

9. Système de désinfection selon l'une quelconque des revendications 3 à 8, **caractérisé en ce que** les moyens d'acquisition sont sous la forme d'un capteur directement intégré dans l'enceinte de désinfection.

## Patentansprüche

1. Desinfektionsverfahren für medizinische Instrumente (1) in einer Desinfektionskammer (4), Einrichtungen zum Erzeugen einer UV-Strahlung zur Desinfektion der Instrumente und einen entsprechenden eingesetzten Sensor (8) umfassend, die dazu angepasst ist, einen Desinfektionszyklus für die Instrumente ablaufen zu lassen, **dadurch gekennzeichnet, dass** jedes Instrument (1) Identifikationsinformationen (5) aufweist, und dass das Verfahren die folgenden Schritte umfasst:
- Erfassen der Identifikationsinformationen des oder jedes Instruments (1) bei seinem Einbringen in die und seiner Entnahme aus der Kammer (4) zu Beginn und am Ende eines Desinfektionszyklus:
- Erfassen von Charakterisierungsinformationen des Desinfektionszyklus, wobei die Charakterisierungsinformationen des Desinfektionszyklus Informationen über eine während des Zyklus abgegebene UV-Dosis umfassen; und
- Zuordnen der Identifikationsinformationen des oder jedes Instruments und der Charakterisierungsinformationen des Desinfektionszyklus, um Rückverfolgbarkeitsinformationen über die Desinfektion des oder jedes Instruments zu generieren, wobei die Rückverfolgbarkeitsinformationen nur dann ausgegeben werden, wenn das entsprechende Instrument (1) bei seinem Einbringen in die und seiner Entnahme aus der Kammer (4) vor und nach dem Desinfektionszyklus jeweils definitiv identifiziert wurde.

2. Desinfektionsverfahren nach Anspruch 1, **dadurch gekennzeichnet, dass** die Identifikationsinformationen durch einen direkt in die Desinfektionskammer integrierten Sensor erfasst werden.

3. Desinfektionssystem für medizinische Instrumente (1), der Bauart mit einer Desinfektionskammer (4), die dazu angepasst ist, einen Desinfektionszyklus für die Instrumente nach einem Verfahren nach Anspruch 1 oder 2 ablaufen zu lassen, Einrichtungen zum Erzeugen einer UV-Strahlung zur Desinfektion der Instrumente umfassend, **dadurch gekennzeichnet, dass** jedes Instrument (1) Identifikationsinformationen (5) aufweist, und dass die Kammer (4) mit Einrichtungen (6) zum Erfassen der Identifikationsinformationen des oder jedes Instruments (1) bei seinem Einbringen in die und seiner Entnahme aus der Kammer (4) zu Beginn und am Ende eines Desinfektionszyklus, mit Einrichtungen (7, 8) zum Erfassen von Charakterisierungsinformationen des Desinfektionszyklus, die Informationen über die während des Zyklus abgegebene UV-Dosis umfasst, die von einem entsprechenden, in die Kammer eingebauten Sensor (8) ausgegeben werden, und mit Einrichtungen (9, 10, 11, 12, 13) zum Zuordnen der Identifikationsinformationen des oder jedes Instruments und der Charakterisierungsinformationen des Desinfektionszyklus, um Informationen über die Rückverfolgbarkeit der Desinfektion des oder jedes Instruments zu generieren, verbunden ist,
und dass die Einrichtungen (9) zum Zuordnen der Informationen dazu angepasst sind, die Rückverfolgbarkeitsinformationen nur dann auszugeben, wenn das entsprechende Instrument (1) bei seinem Einbringen in die und seiner Entnahme aus der Kammer (4) vor und nach dem Desinfektionszyklus jeweils definitiv identifiziert wurde.

4. Desinfektionssystem nach Anspruch 3, **dadurch gekennzeichnet, dass** sich die Identifikationsinformationen (5) jedes Instruments in Form eines Strichcodes darstellen, und dass die entsprechenden Erfassungseinrichtungen (6) der Kammer (4) einen Codeleser umfassen.

5. Desinfektionssystem nach Anspruch 4, **dadurch gekennzeichnet, dass** die Kammer (4) einen Aufhängungsträger für die Instrumente umfasst, und dass der Leser (6) an diesem Träger befestigt ist.

6. Desinfektionssystem nach einem der Ansprüche 3 bis 5, **dadurch gekennzeichnet, dass** die Einrichtungen (7, 8) zum Erfassen von Charakterisierungsinformationen des Desinfektionszyklus Erfassungseinrichtungen für Informationen umfassen, die aus der Gruppe von Informationen ausgewählt sind, die Identifikationsinformationen der Kammer (4) und Zeitstempelinformationen des Desinfektionszyklus umfasst.

7. Desinfektionssystem nach Anspruch 5, **dadurch gekennzeichnet, dass** der UV-Sensor (8) unter dem Träger der Kammer eingebaut ist.

8. Desinfektionssystem nach einem der Ansprüche 3 bis 7, **dadurch gekennzeichnet, dass** die Einrichtungen (9) zum Zuordnen der Informationen mit Visualisierungseineinrichtungen (12) für diese, Speichereinrichtungen (11) für diese und/oder Druckeinrichtungen (13) für diese verbunden sind.

9. Desinfektionssystem nach einem der Ansprüche 3 bis 8, **dadurch gekennzeichnet, dass** die Erfassungseinrichtungen in Form eines direkt in die Desinfektionskammer integrierten Sensors vorliegen.

## Claims

1. Method of disinfecting medical instruments (1) in a disinfection enclosure (4) including means of generating UV radiation for disinfecting instruments and a corresponding implanted sensor (8) suitable for operating an instrument disinfection cycle, **characterised in that** each instrument (1) contains identification information (5) and **in that** the method includes the following steps:
- acquiring identification information on the or each instrument (1) when placed in and removed from the enclosure (4) at the beginning and at the end of a disinfection cycle;
- acquiring characterisation information on the disinfection cycle, the characterisation information on the disinfection cycle comprising information on the UV dose emitted during the cycle; and
- linking identification information on the or each instrument and characterisation information on the disinfection cycle to create traceability information on disinfection of the or each instrument, said traceability information only being produced if the corresponding instrument (1) has in fact been identified when placed in and removed from the enclosure (4) before and after the disinfection cycle respectively.

2. Disinfection method according to claim 1, **characterised in that** the identification information is acquired by a sensor directly integrated in the disinfection enclosure.

3. System of disinfecting medical instruments (1) of the type including a disinfection enclosure (4) suitable for operating an instrument disinfection cycle according to a method according to claim 1 or 2, including means of generating UV radiation for disinfecting instruments, **characterised in that** each instrument (1) contains identification information (5) and **in that** the enclosure (4) is linked to means (6) of acquiring identification information on the or each instrument (1) when placed in or removed from the enclosure (4) at the beginning and at the end of a disinfection cycle, to means (7, 8) of acquiring information on characterising the disinfection cycle comprising information on the UV dose emitted during the cycle produced by a corresponding sensor (8) implanted in the enclosure, and to means (9, 10, 11, 12, 13) of linking identification information on the or each instrument and characterisation information on the disinfection cycle to create traceability information on the disinfection of the or each instrument, and **in that** the means (9) of linking the information are suitable for producing the traceability information only if the corresponding instrument (1) has in fact been identified when placed in and removed from the enclosure (4) before and after the disinfection cycle respectively.

4. Disinfection system according to claim 2, **characterised in that** the identification information (5) on each instrument is in the form of a barcode and **in that** the corresponding acquisition means (6) of the enclosure (4) comprise a code reader.

5. Disinfection system according to claim 4, **characterised in that** the enclosure (4) includes an instrument suspension bracket and **in that** the reader (6) is fixed on this bracket.

6. Disinfection system according to one of claims 3 to 5, **characterised in that** the means (7, 8) of acquiring characterisation information on the disinfection cycle comprise means of acquiring selected information in the group of information comprising identification information on the enclosure (4) and timestamp information on the disinfection cycle.

7. Disinfection system according to claim 5, **characterised in that** the UV sensor (8) is implanted under the enclosure bracket.

8. Disinfection system according to one of claims 3 to 7, **characterised in that** the means of linking the information (9) are linked to means of visualising it (12), means of storing it (11) and/or means of printing it (13).

9. Disinfection system according to one of claims 3 to 8, **characterised in that** the acquisition means are in the form of a sensor directly integrated in the disinfection enclosure.
